# EUROPEAN PATENT APPLICATION

(11) **EP 1 048 304 A1**
(43) Date of publication of application: **02.11.2000**
(21) Application number: 00109237.8
(22) Date of filing: 28.04.2000
(51) Int. Cl.: A61L 27/34

(54) **Neutral coatings**

(30) Priority: 30.04.1999 EP 99108445
(71) Applicant: Novartis AG, 4058 Basel (CH); Novartis-Erfindungen Verwaltungsgesellschaft m.b.H., 1230 Wien (AT); COMMONWEALTH SCIENTIFIC AND INDUSTRIAL RESEARCH ORGANISATION, Campbell ACT 2602 (AU)
(72) Inventor: Griesser, Hans Jörg, The Patch, Victoria 3792 (AU); Kingshott, Peter, 52062 Aachen (DE)
(74) Representative: Becker, Konrad

(57) **Abstract**

The present invention relates to a composite material, comprising a bulk material and an antifouling hydrophilic coating wherein said anti-fouling coating consists in particular of a neutralized polysaccharide. Articles, in particular biomedical devices, comprising such composite materials are described too.

## Description

The present invention relates to a composite material, comprising a bulk material and an antifouling hydrophilic coating wherein said anti-fouling coating consists in particular of a neutralized polysaccharide. Articles, in particular biomedical devices, comprising such composite materials are described too.

The irreversible adsorption and subsequent denaturation of proteins ("fouling") onto biomedical devices initiates various adverse consequences that need to be overcome by the design of improved biomaterials. In the case of contact lenses for instance, fouling not only necessitates cleaning and limits the time that a contact lens can be worn, but more importantly the adsorbed proteins initiate adverse events. For instance, surface-adsorbed proteins such as fibronectin can serve as ^{"}platforms^{"} onto which bacteria attach. In the case of blood-contacting devices such as vascular grafts and synthetic heart valves, the irreversible adsorption of proteins such as fibrinogen leads to platelet activation, blood clotting, and thrombosis. Other biomedical devices, such as transdermal and urinary catheters, can serve as sites for bacterial infections. Again, it is thought that host proteins adsorbing onto the device promote bacterial attachment.

Clearly, thus, there is a need for biomaterials that possess the ability to considerably reduce, or totally eliminate, the irreversible adsorption of proteins. This need has been recognized for many years in the biomaterials literature and there are many publications and patents that describe "non-fouling" or "anti-fouling" surfaces or coatings. On closer inspection, however, it transpires that the researchers either have used methods to measure amounts of adsorbed proteins which do not possess state-of-the-art sensitivity, or that they use comparative descriptions by comparing their new surface/coating with an established material (often a highly adsorbing but widely used known material such as polyethylene or PVC is chosen in order to dramatize the effect !). Typically "non-fouling" or "anti-fouling" surfaces or coatings have been measured to adsorb protein amounts of the order of 300 ng/cm², which corresponds approximately to a monolayer of proteins. Such coverage is typically achieved within 1-2 hours or less. Thefore, the need still exists to generate surfaces or coatings that can minimize protein adsorption for extended periods of time.

EP 613'381 discloses multilayer materials comprising polysaccharide coatings. Such coatings have been found to be relatively low fouling compared with known, conventional hydrogels such as poly-HEMA, and have been useful for the fabrication of contact lens coatings with good clinical performance (H.J. Griesser et al., Polysaccharide Coatings for Contact Lenses, Polymeric Materials Science and Engineering, 76, 79 (1997)).

The present invention improves upon this and other prior art by disclosing coatings, in particular modified polysaccharide coatings that further reduce the amounts of proteins that can adsorb onto them. The concept and chemical method for reducing protein adsorption as exemplified herein on polysaccharide coatings is, however, also transferable to other, similar chemical coating structures and is applicable to reducing protein fouling on various biomaterials.

The present invention is therefore directed to the subject matter as disclosed in claim 1. The neutralizing agent is an organic base in case of wettability providing groups -COOH or - SO₃H, or it is an organic acid in case of wettability providing groups -NH₂. The term "at least partially" as used herein means preferably that at least 20 % of such groups are modified, more preferred that at least 50 % of such groups are modified , and most preferred that at least 80 % thereof are modified, all these numbers being calculated on a molar basis.

The invention is also directed to a process for improving the non-fouling properties of a hydrophilic coating on a bulk material characterized in that wettability providing groups in said coating selected from -COOH or -SO₃H are treated at least partially with an organic amine, or in that wettability providing groups in said coating which are -NH₂ groups are treated at least partially with an organic acid.

In the case of contact lens fouling, lysozyme is a major foulant protein. In some cases it even is the only protein to constitute the first monolayer of adsorbed protein that conditions the contact lens surface for further adsorption of subsequent layers of various proteins and lipids. Lysozyme associates with negatively charged chemical groups of contact lens materials. Thus, contact lens coatings fabricated from negatively charged polysaccharides such as hyaluronic acid and chondroitin sulfate, while highly wettable, and relatively low fouling compared with much of the prior art, do not possess the ultimately desirable optimal resistance to lysozyme adsorption.

Typical polysaccharides are selected from hyaluronic acid, deacylated hyaluronic acid, chitosan, chitin 50, fucoidan, carrageenans, chondroitin sulfate, dextran, blue dextran, aminated dextran, carboxyalkylated dextran, glaktomannan, pullulan, glycosaminoglycan, heparin, agarose, curdlan, pectin, pectic acid, xanthan, hydroxypropyl cellulose or chitosan, carboxymethyl cellulose or chitosan, emulsan, laminaran, inulin, pustulan, scleroglucan, schizophyllan and mucopolysaccharides.

Preferred polysaccharides are selected from hyaluronic acid, deacylated hyaluronic acid, chondroitin sulfate, dextran, blue dextran, aminated dextran and carboxyalkylated dextran.

The term alkyl denotes a radical with up to 18 carbon atoms, preferably 12 carbon atoms and in particular up to 7 carbon atoms.

The present invention relates in particular to polysaccharide coatings whose charged groups have been blocked by suitable chemical reactions to produce uncharged, so called neutral coatings. Such coatings possess much superior resistance to protein adsorption, accumulating much less than a monolayer of total protein material in 2 hours of immersion in artificial tear fluid, as compared with the known polysaccharide coatings disclosed in EP 613'381, which accumulate a monolayer amount of proteins (of the order of 300 ng/cm2) within that time and in some instances within much shorter times (see examples below).

The terms uncharged groups and/or coatings and neutral groups and/or coatings are used within the terms of this invention as synonyms. Accordingly, the terms charged and non-neutral are used as synonyms too.

The neutral coatings of the present invention are produced by the covalent anchoring of polysaccharide molecules onto a bulk material, which is preferably a bulk biomaterial. As described in EP 613'381, the polysaccharide molecules can either be attached directly onto the bulk material by an interfacial reaction that produces one or more covalent bonds between a polysaccharide molecule and the surface of the bulk material, or they can be attached via an intermediate thin interfacial bonding layer. The direct attachment requires either that the bulk material possesses suitable surface chemical groups for covalent interfacial reactions, or that such reactive groups are introduced by a surface treatment known and described in the art, such as an ammonia gas plasma (radio frequency (rf) glow discharge) treatment. Often, however, the application of an intermediate thin interfacial bonding layer is preferable as this method has been found to often confer better coating quality (uniformity and reproducibility) and adhesion of the polysaccharide molecules. This has all been disclosed in detail in EP 613'381. The present invention is characterized by an additional subsequent step which enhances fouling resistance.

In more detail, the highly wettable but not optimally fouling-resistant polysaccharide coatings fabricated for example in accordance to EP 613'381 are then modified by a further chemical reaction that converts charged chemical groups to neutral groups. For instance, carboxylate groups (which are mostly deprotonated at the pH values of biological media such as tears and blood) of e.g. a hyaluronic acid coating are typically neutralized by reaction with an amine such as for instance ethanolamine. This converts the carboxylate groups to amide groups and further provides an additional hydroxyl group which enhances wettability of the coating. The latter (the hydroxyl group) is, however, not necessary for the present invention to work. The key step is the conversion of carboxylate groups to a group that is uncharged at a physiological pH value. Such groups are typically selected from an amide and an ester. Amide groups are particularly preferred. Amide groups can be generated for example by the well-known N-ethyl-N'-(2-dimethylaminopropyl)-carbodiimide hydrochloride (EDC)/ N-hydroxysuccinamide (NHS) reaction.

However, the invention is not restricted to a polysaccharide. Other carboxylate-containing coatings can be fabricated, for instance by the covalent attachment onto biomaterials of 90:10 polyacrylamide/polyacrylic acid copolymer and the like. This copolymer is attached covalently onto surfaces containing amine groups, e.g. by the EDC/NHS reaction, which couples carboxylic acid groups of the copolymer onto the surface amine groups, under acidic conditions. After the pH is raised to values applicable to physiological usage, however, remaining carboxylate groups exist in and on the surface of the coating, since both for steric reasons and for reasons of surface amine density it is impossible to react all carboxylic acid groups with amine groups. These remaining carboxylate groups can then be neutralized e.g. by the EDC/NHS reaction.

It should not be concluded that the present invention is applicable only to fouling events dominated by lysozyme. Negatively charged proteins can adsorb onto negatively charged (eg carboxylated) surfaces and coatings too. It is thought that this occurs by way of ionic bridging, that is, with bivalent ions such as Ca²⁺ as intermediates. Thus, the neutralization of carboxylate groups is expected to be beneficial even when fouling is not dominated by lysozyme.

Nor is the concept of surface/coating charge neutralization restricted to carboxylate groups. Charged surfaces and coatings can comprise other groups, such as amines. Amine groups are positively charged at physiological pH values. Thus, negatively charged proteins can associate ionically with such charged amine surfaces, and positively charged proteins also may bind via ion bridging. However, if amine groups are converted into neutral groups e.g. by known reactions such as acetylation, the charge on such surfaces and coatings can be neutralized. An example is a coating made by covalently anchoring aminodextran onto aldehyde or carboxylic acid surfaces. The attachment consumes some of the amine groups but the remaining amine groups of the aminodextran coating present positive charges to the biological medium. Neutralization of positively charged groups is also an object of this invention.

The much reduced tendency for protein adsorption of the coatings of this invention enables the manufacture of contact lenses suitable for longer wear times with fewer clinical complications, due to the reduction in adverse consequences such as bacterial colonization that is expected to be consequent on reduced fouling.
The coatings of the present invention can be applied onto various polymeric materials ("bulks") such as the extended wear contact lens (EWCL) materials of perfluoropolyether or silicone containing compositions.

The objective of the invention is to provide coatings that have a reduced rate of fouling by proteins and thus a reduced rate of adverse biomedical consequences.

This problem has been solved by neutralizing the charged groups of surfaces or coatings. The term neutral coating structures should be understood within the terms of this invention in the microscopic sense, namely of not having any charged groups. The macroscopic neutrality arising from cancellation of charges in zwitterion structures shall not relate to said neutralization.

### Description of the Drawings

**Figure 1** shows XPS analysis of the accumulation of lysozyme on a heptylamine plasma polymer coating. The initial datum (time zero) represents the nitrogen content of the heptylamine plasma polymer coating, and the increase in the nitrogen content represents further nitrogen due to lysozyme.
**Figure 2** contains data from XPS analyses of polysaccharide coatings as fabricated ("control") and after 1 hour of immersion in artificial tear fluid. CM (1/30) is a carboxymethylated dextran, wherein the ratio of carboxymethyl groups to anhydroglucopyranoside ("dextran") units is 1: 30; CM (1/13) is a carboxymethylated dextran, wherein the ratio of carboxymethyl groups to "dextran" units is 1 : 13; CS = chondroitin sulphate; HA = hyaluronic acid; HA / EA means hyaluronic acid which is at least partially modified with ethanolamine; OD = oxidized dextran.
**Figure 3** shows schematically the coupling of hyaluronic acid (HA) to the amino groups contained on the surface of fluorinated ethylene propylene copolymer (Teflon¨ FEP) which is plasma coated with n-heptylamine (n-ha pp), whereby said coupling is carried out via an activated ester (N-hydroxysuccinimide ester).
**Figure 4** shows schematically the modification (neutralization) of the remaining carboxylic groups of hyaluronic acid (HA) with an organic base (here ethanolamine).
**Figure 5** shows the structures of hyaluronic acid (HA) (top) and chondroitinsulphate (CS) (bottom).
**Figure 6** shows XPS analysis of the accumulation of ATF proteins on a heptylamine plasma polymer coating.

### Determination of Protein Absorption

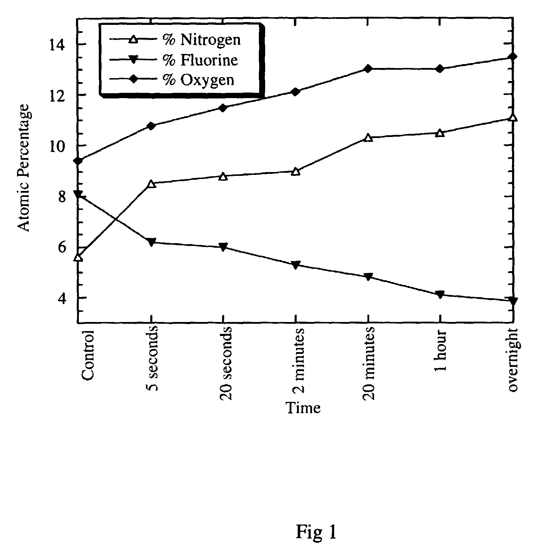

The accumulation of proteins on surfaces and coatings can be measured by X-ray photoelectron spectroscopy (XPS), by the increase in the N signal indicative of protein amounts, and by the reduction in the signals of the biomaterial as it becomes covered by proteins. Figure 1 shows XPS analysis of the accumulation of lysozyme on a heptylamine plasma polymer coating. The initial datum (time zero) represents the nitrogen content of the heptylamine plasma polymer coating, and the increase in the nitrogen content represents further nitrogen due to lysozyme. The samples were rinsed thoroughly after immmersion in protein solutions, and thus the amounts of proteins detected represent tightly, i.e. irreversibly, adsorbed proteins. Although one may have expected no lysozyme adsorption onto such an amine-containing surface, clearly fouling by lysozyme does occur, to a level of approximately one monolayer coverage (∼ 220 ng/cm2) within 20 minutes; the amount calculated from these XPS data has been verified by a quartz crystal microbalance method. Thus, other strategies than "like charges" must be implemented to prevent adsorption of proteins of a given charge.

For polysaccharide coatings, analogous kinetic plots of protein accumulation were obtained. Some of the coatings disclosed in EP 613'381 surprisingly showed similar rates of fouling by proteins as the above polymeric coating although the polysaccharide coatings were extremely hydrophilic and had a high affinity for water as shown by their ability to tenaciously retain a water layer. Most remarkably, a coating consisting of chondroitin sulfate, which was the most hydrophilic coating tested according to contact angle data, was found to suffer from a faster rate of fouling than many other coatings.

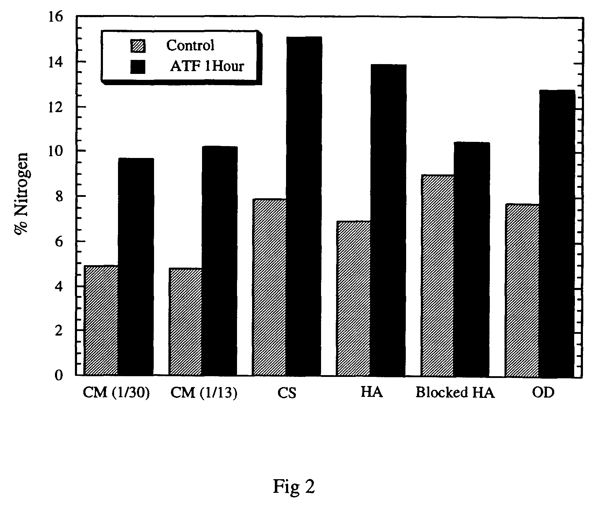

Figure 2 contains data from XPS analyses of polysaccharide coatings as fabricated ("control") and after 1 hour of immersion in artificial tear fluid. Again the amounts of proteins represent only irreversibly bound proteins. The finite nitrogen level of the control samples is due to the nitrogen-containing interfacial bonding layer upon which the polysaccharides were immobilized. Thus, as for Figure 1, it is the increase in the N signal over the control that is a measure of the amounts of adsorbed proteins.

Clearly, the negatively charged coatings, for example carboxymethylated dextrans (CMs), chondroitin sulphate (CS), hyaluronic acid (HA), adsorb considerable amounts of proteins within one hour; CS and HA adsorb to monolayer coverage. Oxidized dextran (OD) is a coating that possesses no charge in the polysaccharide itself but the covalent linkage is an amine bond and thus some positive charging exists. Clearly, the coatings with a smaller amount of charge (CMs, OD) adsorb smaller amounts of protein within a given time.

However, it is the ethanolamine-blocked hyaluronic acid (EA/HA) coating that shows the best performance by far; it adsorbs much less than a monolayer within one hour.

It is difficult to ascertain whether the blocking reaction (converting carboxylate groups to hydroxyethylamides) had occurred to 100% efficiency, and it is possible that a small amount of negatively charged carboxylate groups remained in the HA/EA coating. However, clearly the benefit of reducing the number of charged groups is evident.

### Utility

The invention is not restricted to contact lenses. Charge interactions are very important in many other biomaterials applications, and the fabrication of neutral coatings is expected to lead to reduced fouling and thus reduced incidence of adverse biomedical consequences in many applications. A prime target for the coatings of the present invention is for blood contacting applications (vascular grafts, heart valves, dialysis membranes), where reduction of fibrinogen adsorption is highly beneficial.

The coatings of this invention are also useful as antibacterial coatings for applications such as indwelling catheters.

Another area where uncontrolled protein adsorption reduces the efficacy of devices is in the biosensors field. A charge-neutralized coating carrying specific recognition entities such as antibodies would be expected to provide benefits in enhanced signal-to-noise ratios and thereby lower detection limits and more reliable assays.

### Examples:

### 1.Plasma Polymer Surfaces.

Fluorinated ethylene propylene copolymer (Teflon¨ FEP) of 25mm thickness and 12.7 mm width was purchased from Du Pont and was used as the base substrate for the preparation of the functionalised surfaces. FEP has a number of distinct advantages over other materials, such as polystyrene and glass, that have been used as model substrates. It does not readily adsorb hydrocarbon contaminants from the atmosphere, and therefore does not require extensive cleaning procedures. The clean surface also ensures that a well defined structure exists and elemental compositions, determined by XPS, are close to the theoretical composition, assisting in the accurate calculation of overlayer thicknesses. All coatings used in this study do not contain fluorine, and therefore, the attenuation of the F 1s signal of the FEP substrate can be used to determine the thickness of the overlayers (see equations in text). The smooth topography of FEP, as determined by scanning tunneling miscroscopy (STM), facilitates its use in angular resolved XPS (ARXPS) studies.

### 2. Plasma Apparatus.

FEP substrates were coated with plasma polymer films using technology based on standard radio-frequency (rf) glow discharge. Plasma polymerisation was performed in a custom built reactor. The reactor chamber consists of a vertical glass cylindrical of diameter 16.5 cm and height 34 cm. The glow discharge is generated between copper electrodes 11 cm apart. The base electrode is circular with a diameter of 9.5 cm, upon which the FEP strips are placed for coating. The upper electrode is U-shaped (17 cm long and 8 cm wide) and made from copper rods. The process vapor ("monomer") gas inlet is situated between the two vertical electrode rods in the top plate, which is used to seal the chamber. The process vapor feed line consists of a pressure control valve, to control the flow rate, and a bellows which is attached to a round bottom flask containing the monomer liquid. The exhaust outlet is situated in the base plate (PTFE) under the base electrode. There is also a separate gate valve at the bottom of the chamber for venting the entire chamber. The rf glow discharge is powered by a radio frequency power generator (ENI, Model HPG-2), operating at a variable frequency in the range 125-375 kHz. A Javac oil sealed high vacuum pump is used to evacuate the system. A pressure transducer ( MKS Baratron, Model 127A) was used to accurately measure the pressure in the chamber.

### 4.2.3 Plasma Polymerisation Conditions.

The chamber was pumped down to a base pressure of 0.01 mBar, or less, prior to the monomer gas being introduced. n-Heptylamine (Aldrich Chemical Co., ³99 %) monomer was used to coat the FEP substrate with a thin polymeric coating containing amine groups for subsequent polysaccharide immobilization. Typical plasma deposition conditions used were: power, 20 Watts; deposition pressure, 0.12 mbar; frequency, 200 kHz. In order to obtain thin coatings suitable for thickness measurements by attenuation of the XPS F 1s signal (thickness < 12 nm), deposition times between 3 and 5 seconds were used. The resultant coatings were measured to be 3-4 nm thick. All other plasma depositions were performed for 20 seconds.

### 3. Polysaccharide Coatings.

Chondroitin Sulphate (CS), NaCNBH3 (90 %), and N-ethyl-N'-(2-dimethylaminopropyl)-carbodiimide hydrochloride (EDC) were all purchased from Sigma and used without further purification. NalO4 (99.8 %+) and N-hydroxysuccinamide (NHS) were supplied by Aldrich and used as received. Carboxymethyl dextrans (CMDs) were prepared by using the reaction between bromoacetic acid and dextran, and used without further purification. Similarly, dextran (Sigma, MW 71,400 Da) was carboxymethylated by reaction with bromoacetic acid. Carboxymethyldextrans (CMDs) with different ratios of carboxyl groups to anhydroglucopyranoside ("Glc") ring units were prepared by using varying ratios of dextran to bromoacetic acid in order to obtain different extents of substitution.

### 3.1. Preparation of Carboxymethyldextrans:

10g of dextran was dissolved in 50 ml of 2M NaOH containing either 0.125 M, 0.25 M or 1M bromoacetic acid. The solution was stirred overnight, dialysed, and lyophilised. The degree of carboxylation was determined by back titration and by NMR spectroscopy. The experimentally determined ratios were 1:2, 1:14 and 1:30. The different substitution ratios allowed the fabrication of coatings with different densities of surface attachment and thus different coating microstructures.

MilliQ H₂O (Millipore system, 18.2 M½/cm) was used to prepare all solutions and to rinse all cleaned glassware.

### 4. CS, HA, and CMDs.

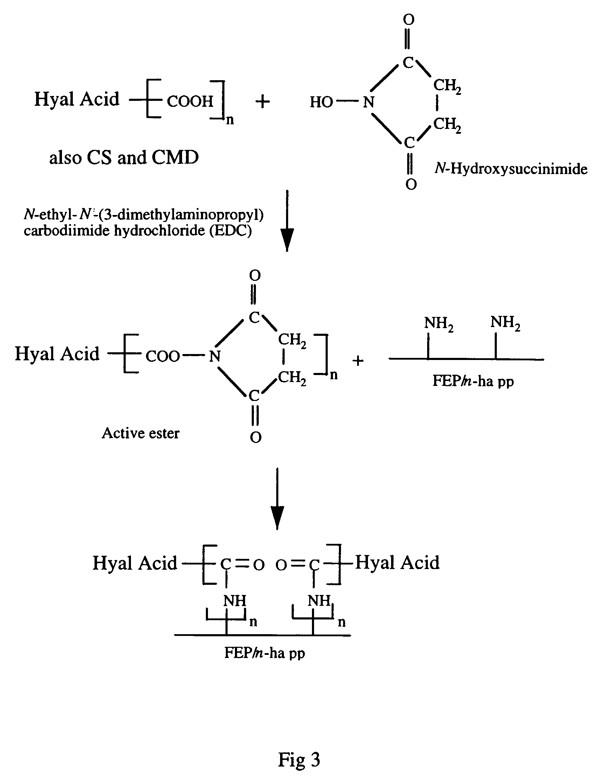

Freshly deposited n-heptylamine plasma polymer (n-ha pp) substrates were immersed in 1 mg/mL aqueous solutions (ca. 18 mL) of the appropriate polysaccharide and shaken for 1 hour, to allow physical adsorption of the polymer to reach a plateau. The carboxyl groups present in the polysaccharides were activated to form N-hydroxysuccinimide esters by addition of a 2.0 mL solution of N-ethyl-N'-(2-dimethylaminopropyl)carbodiimide hydrochloride (EDC, 19.2 mg/mL) and N-hydroxysuccinamide (NHS, 11.5 mg/mL). The reaction scheme for hyaluronic acid (HA) immobilisation to n-ha pp is depicted in Figure 3. The substrates were shaken overnight, rinsed three times with H₂O and visually checked for water wettability.

### 5. Hyaluronic acid + Ethanolamine Neutralization.

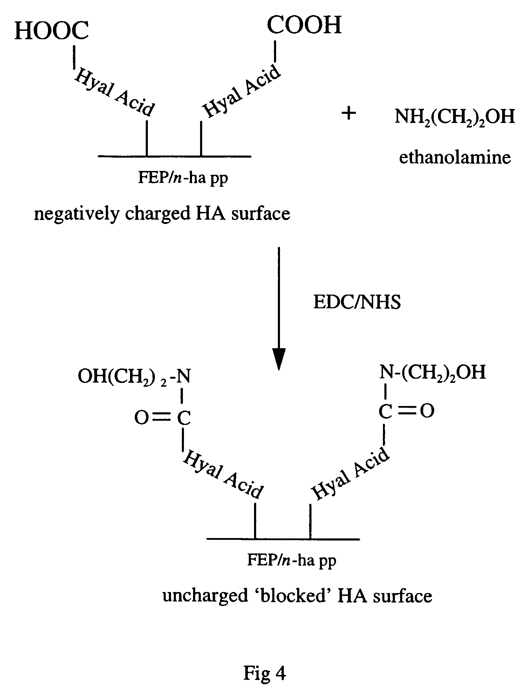

Strips of FEP / n-happ / HA substrate are immersed in a 6 M aqueous solution of ethanolamine (NH₂(CH₂)₂OH) for 1 hour, followed by reaction with EDC/NHS, as depicted in Figure 3 , and shaken overnight, followed by rinsing three times with H₂O, and visually checking for water wettability. The reaction is designed to block the residual carboxyl groups in the HA surface that potentially adsorb oppositely charged proteins, as outlined in Figure 4.

### 7. Preparation of Protein Adsorption Solution.

Lysozyme (chicken egg, 95%), lactoferrin (bovine colostrum, 90%), α-acid glycoprotein (bovine, 99%), γ-globulins (human, 99%), human serum albumin, (HSA, 97-99%), bovine submaxillary mucin, (BSM, Type 1-S), trypsinogen (bovine pancreas), myoglobin (horse heart, 95-100 %), cholesteryl linoleate (98+% ), triolein (99%), oleic acid propyl ester (99%), dicaproin (99%, mixed isomers), undecylenic acid (Na salt, 98%), and cholesterol (99+%) were purchased from Sigma and used without further purification. Dodecyl trimethyl ammonium bromide (DTAB, 98%, BDH Chemicals) and linalyl acetate (97%, Aldrich) were used without further purification. NaCl, KCl, NaHCO3, lactic acid, CaCl2, and NaH₂PO₄ H₂O were of analytical grade. 3-(N-morpholinopropane)-sulphonic acid (MOPS) buffer was purchased from Calbiochem. Analytical grade toluene, ethanol and n-hexane were purchased from BDH Chemicals.

### 8. Preparation of ATF.

Artificial tear fluid (ATF) was prepared in accordance to prior art. Table 1 is a list of all the ATF components. The preparation procedure required the following series of steps:
Step 1. Buffer: A stock solution (ca. 250.0 mL) of buffer containing all of the salts was prepared prior to the dissolution of proteins and lipids. CaCl₂ was added last to the buffer solution to prevent the precipitation of CaCO₃ which is insoluble at low ionic strength.
Step 2. Stock Lipid Mixture: Oleic acid propyl ester (0.30 g), triolein (0.40 g), dicaproin (0.08 g), and linalyl acetate (0.50 g) were combined and mixed by shaking. Cholesterol (0.012 g), cholesteryl linoleate (0.18 g), and lipid mixture (0.408 g) were then mixed by applying shaking and gentle heating over a water bath. The temperature was kept at a minimum (<50°C) to prevent volatilisation of the lipids. The stock lipid mixture was stored desiccated under nitrogen at -18°C.
Step 3. Mucin-Lipid Mixture (ca. 200 mL): The final lipid mixture (0.016 g, Step 2) and mucin (0.30 g) were homogenized by pressing the solid and liquid together with a pestle in a small beaker (ca. 10 mL). Three 1.0 mL portions of buffer were added to assist with the micellisation of mucin and lipids. Frictional heat generation was minimised to prevent thermal denaturation of the mucin and volatilisation of the lipids. Undecylenic acid (0.003 g) was dissolved in 1.0 mL of buffer, and 0.1 mL of this solution was added to the mucin-lipid mixture. The final mucin-lipid mixture was made up to 200.0 mL in a volumetric flask. Any residual mixture was thoroughly rinsed from the mixing beaker in the process.
Step 4. Addition of Proteins: The final ATF was prepared in batches of 10.0 mL to ensure that it would be consumed before deterioration or microbial growth. Each protein, viz., lysozyme (95 mg), lactoferrin (95 mg), α-acid glycoprotein (25 mg), human serum albumin (10 mg), and γ-globulins, was added to the mucin-lipid mixture (ca. 50.0 mL) in 20 mg portions or less to fully solubilize them and prevent aggregation and bubble formation.

**Table 1**

| The Structure of Artificial Tear Fluid (ATF). | | |
|---|---|---|
| **Component** | **[ ]/mg/mL** | **Molecular Weight** |
| Proteins and Glycoproteins | | |
| lysozyme | 1.9 | 14304 |
| lactoferrin | 1.8 | 82000 |
| α-acid glycoprotein | 0.5 | 44100 |
| human serum albumin (HSA) | 0.2 | 69000 |
| mucin | 0.15 | ∼880000 |
| γ-globulins | 0.1 | 156-161000 |

| Lipids | | |
|---|---|---|
| cholesteryl linoleate (C₄₅H₇₆O₂) | 0.024 | 649.1 |
| linalyl acetate (C₁₂H₂₀O₂) | 0.02 | 196.3 |
| triolein (C₅₇H₁₀₄O₆) | 0.016 | 885.4 |
| oleic acid propylester(C₃₆H₇₀O₂) | 0.012 | 534.9 |
| dicaproin (C₁₅H₂₈O₅) | 0.0032 | 288.4 |
| undecylenic acid (C₁₁H₂₀O₂) | 0.003 | 184.3 |
| cholesterol (C₂₇H₄₆O) | 0.0016 | 386.7 |

| Salts | | |
|---|---|---|
| NaCl | 6.626 | |
| KCl | 1.716 | |
| NaHCO₃ | 1.374 | |
| lactic acid | 0.27 | |
| CaCl₂ | 0.147 | |
| NaH₂PO₄.H₂O | 0.1 | |

| Buffer | | |
|---|---|---|
| 3-(N-morpholinopropane-sulphonic acid) (MOPS) pH-7.4 with 1N NaOH | 4.18 | |

The ATF was kept at 4°C overnight and the pH adjusted to 7.40 with 1N NaOH, before being frozen at -18°C. Prior to the adsorption experiments the ATF was thawed to room temperature (ca. 22°C). The freeze-thaw process of protein solutions is crucial in preventing their deterioration.

### 9. Adsorption Methodology.(Adsorption Methodology involving ATF)

The adsorption behaviour of all polysaccharide (PS) coatings towards ATF is compared by immersing pieces of the substrates in 2.0 mL ATF solution for 1 hour, followed by rinsing three times with H₂O, before storing under H₂O until analysis. In addition, pieces of OD and CS substrates are immersed in ATF solution for periods ranging from 5 seconds to overnight adsorption (ca. 16 hours), to compare the time dependence of adsorption. The OD substrate is also exposed to a modified ATF solution depleted of lysozyme for the same time regime. The CS substrate is immersed in the same solution for 1 hour.

### 10. Immobilisation of HA, CS, and CMDs.

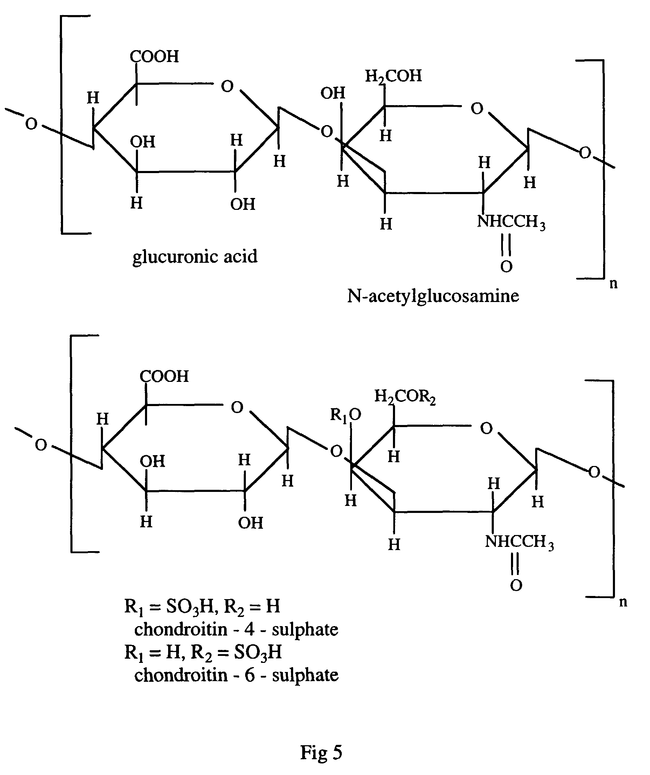

Wettability or hydrophilisation is one the essential surface criterion necessary for the developement of an extended wear contact lens. As an alternative to OD four other polysaccharides are utilized in an attempt to meet this criterion. The CMDs are prepared by converting a controllable fraction of the hydroxyl groups of dextran to carboxymethyl groups with bromoacetic acid. Two different CMDs are chosen, one with a CM/sugar ratio of 1/13 and one with a CM/sugar ratio of 1/30. The rationale is to vary the number of attachment points per monomer of dextran and produce coatings that varied in protein rejecting ability based upon the degree of 'floppiness' at the immediate interface. Attachment of both HA and CS takes advantage of the chemistry of the native structure, eliminating the need for an additional modification step. The structures of both are presented in Figure 5. Each monomer unit of both chondroitin sulphate and hyaluronic acid consists of a glucuronic acid sugar unit containing one residual carboxylic acid functional group.

Figure 3 outlines the attachment chemistry for the immobilisation of HA to the n-ha pp surface, and can also be applied to the CS and CMD surfaces. After allowing the polysaccharide sufficient time to physically adsorb to the n-ha pp surface (ca. 1 hour) the carboxyl groups are reacted with EDC/NHS, generating active N-hydroxysuccinimide esters, which are subsequently displaced by the primary amines groups on the n-ha surface, forming a covalent amide bond with the polysaccharide molecules.

All the polysaccharides grafting using the reaction scheme in Figure 3 exhibited excellent visual water wettabilities when held in the vertical position. Occasionally after the preparation a surface would show some water breakup and was discarded, with the grafting regarded as being patchy. Table 2 summarises the XPS elemental compositions of the three polysaccharides grafted using the EDC/NHS reaction mechanism, and also the data for each surface after immersion in ATF for 1 hour.

Firstly, upon attaching CS, the oxygen content increases from 4.0 % for the n-ha pp surface to 18.5 % and the carbon content decreases from 80.4 % to 68.7. This change is consistent with expectations based on the C and O contents of chondroitin sulphate but since the composition of the coated sample is not identical to that of pure CS, it is immediately evident that, as for the OD coating, the thickness of the CS coating is substantially less that the XPS sampling depth. The S content of 0.8 % for the grafted surface provides further evidence to the successful reaction since the underlying n-ha surface does not contain any sulphur whereas the CS contains two S atoms per monomer unit, in the form of sulphonate (Figure 5). There is also an increase in N content from 6.6 % for the control to 7.9 % for the grafted surface due to the N-acetylglucosamine group (-NHCOCH₃) of CS.

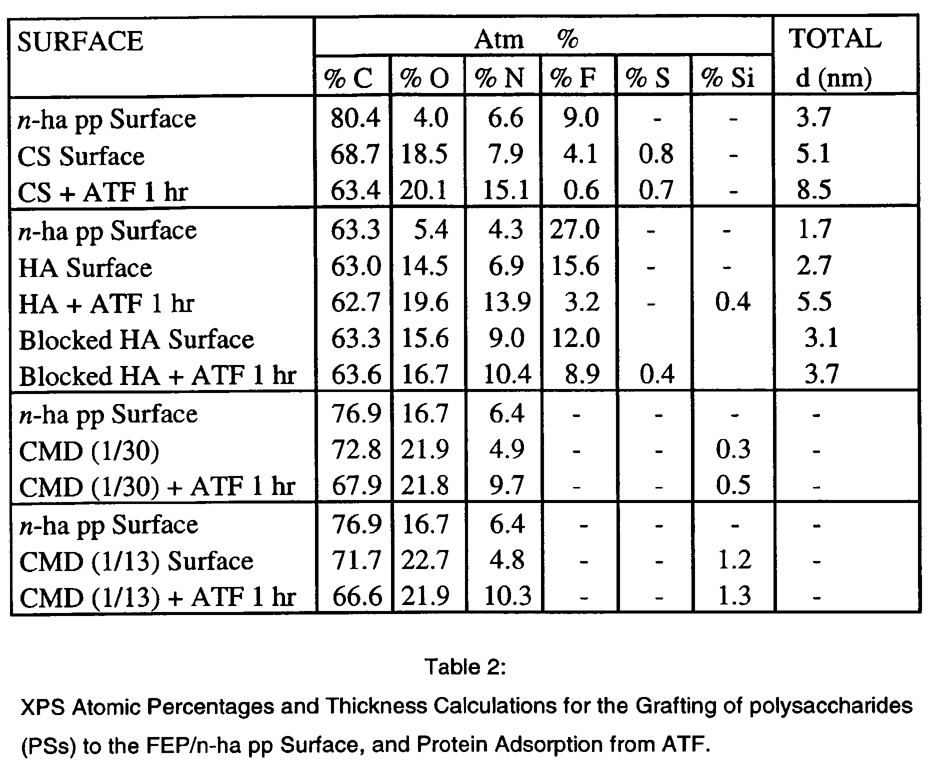

For instance when attaching CS, there is an increase in thickness of 1.4 nm due to the grafted layer as calculated from the increased attenuation of the F 1s signal relative to the n-ha pp control. The thickness is not a true representation of the actual thickness in an aqueous environment because the CS polymer is substantially hydrated in contact with aqueous media whereas it is dehydrated in the vacuum environment of the XPS analysis.

### 11. Protein Adsorption to the polysaccharides from ATF.

Table 2 lists all of the XPS data for adsorption of protein from ATF after 1 hour to the five polysaccharides surfaces, and Figure 2 is a comparative plot of the N content for each surface, including the HA blocked surface, before and after immersion in ATF. All polysaccharide surfaces show a significant increase in nitrogen signal after immersion in ATF for 1 hour corresponding to the adsorption of protein.

The CS and HA surfaces exhibits the highest level of protein adsorption from ATF solution of all the polysaccharide surfaces tested. The nitrogen content of the CS surface increases from 7.9 % for the control to 15.1 % after 1 hour immersion in ATF solution, whereas the HA surface N content increases from 6.9 % to 13.9 %. These increases in the nitrogen content are consistent with approximately monolayer coverage by adsorbed proteins. The high number of carboxyl groups in the large polymer of HA suggests that not every group, once activated, will be able to covalently attach to the amine groups of the n-ha pp surface due to steric hindrance. Therefore, there is likely to be a substantial number of remaining carboxylate groups in the HA coating, which confer a negative charge at pH 7.4. These remaining carboxylate groups were intended to be blocked with ethanolamine, in order to study whether the negative charge of the HA coating is responsible for it adsorbing proteins from ATF.

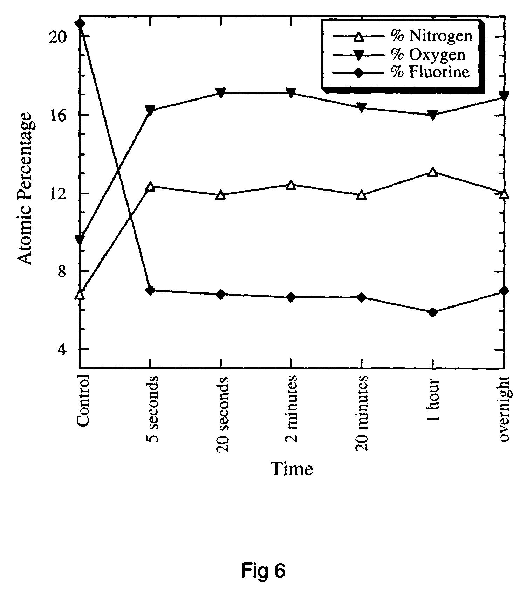

A deactivation (neutralization) step of the residual unreacted carboxyl groups of the HA surface with ethanolamine was undertaken in an attempt to reduce the amount of protein adsorbed from ATF. XPS data suggests (Table 2, Figure 6) that the reaction was successful as indicated by the increase in N content from 6.9 % to 9.0 %. The O content also increased from 14.5 % to 15.6 %, and the thickness of the modified hyaluronic acid surface was calculated to be 3.1 nm compared to 2.7 nm for the original surface providing further evidence that the blocking agent was coupled to the HA surface.

The modified HA surface was immersed in ATF for 1 hour and the XPS data suggest that there is a significant reduction in the amount of protein adsorbed to the surface, with the nitrogen content increasing from 9.0 % to 10.4 %. This is compared to increase from 6.9 % to 13.9 % for the unmodified surface. The thickness of the unmodified surface increased from 2.7 nm to 5.8 nm, whereas the blocked surface only increased from 3.1 nm to 3.6 nm. A significant proportion of the residual negatively charged carboxyl groups in the HA matrix have been successfully blocked since there is a substantial decrease in the level of protein adsorption. That is, there is a reduction in electrostatic attraction of proteins from ATF, such as lysozyme. However, the efficiency of the blocking is unknown and the protein adsorption may be caused by some unblocked carboxyl groups or another interfacial interaction with the proteins of ATF.

## Claims

1. A composite material with a wettable surface being significantly less susceptible to protein absorption (fouling), which composite material comprises a bulk material and a hydrophilic coating, said hydrophilic coating being covalently attached to reactive groups at the surface of said bulk material by conventional technique, either directly or indirectly via the functional groups of an oligofunctional organic linker, wherein said hydrophilic coating comprises a multitude of pH-susceptible wettability providing groups which are selected from the group consisting of -COOH, -NH₂ and -SO₃H, characterized in that said wettability providing goups are modified at least partially with a neutralizing agent which neutralizing agent is selected from an organic acid and/or an organic base.

2. Material of claim 1, wherein the reactive groups at the surface of said bulk material are either inherently present or wherein said reactive groups have been attached to the surface of said bulk material via a plasma surface treatment.

3. Material of claim 1, wherein said hydrophilic coating is selected from the group consisting of a polysaccharide, a (meth)acrylic acid homopolymer and a (meth)acrylic acid copolymer.

4. Material of claim 1, wherein said oligofunctional organic linker is a bis-oxirane, a diisocyanate, a dicarboxylicc acid chloride, a ditosylate, a dimesylate or an epihalohydrin.

5. Material of claim 1, which is a biomedical device, an ophthalmic device or a contact lens, preferably an ophthalmic device or a contact lens, and more preferably a contact lens.

6. Material of claim 1, wherein said neutralizing acid is selected from a carboxylic acid such as formic acid; linear or branched alkane carboxylic acid such as acetic acid, propionic acid; aryl carboxylic acid such as benzoic acid; linear or branched alkane sulfonic acid such as methane sulfonic acid; aryl sulfonic acid such as benzene sulfonic acid and toluene sulfonic acid.

7. Material of claim 1, wherein said neutralizing base is selected from a secondary amine such as linear or branched dialkyl amine which is unsubstituted or substituted by one or more hydroxy groups in an alkyl chain; and a primary amine such as linear or branched alkyl amine which is unsubstituted or substituted by one or more hydroxy groups in said alkyl chain, wherein alkyl denotes a radical with up to 18 carbon atoms, preferably 12 carbon atoms and in particular up to 7 carbon atoms.

8. Material of claim 1, wherein said hydrophilic coating is a polysaccharide and is selected from dextran, carboxymethyl dextran, chitosan, hyaluronic acid, mucin, fucoidan, chondroitin sulfate and glucosamin.

9. Material of claim 1, wherein said modification of said wettability providing groups consists of a multistep reaction.

10. Use of a neutralized wettability providing group in the reduction of the surface protein adsorption of a hydrophilic polymeric coating in accordance to claim 1.

11. Use of an organic base and/or an organic acid in the neutralization of a wettability providing group comprised in a hydrophilic coating in accordance to claim 1.

12. Use of claim 11, wherein said neutralization is a charge neutralization and wherein said wettability providing group is a protonated or deprotonated ioinized group.

13. A process for the manufacture of a composite material with a wettable surface being significantly less susceptible to protein absorption (fouling) according to claim 1, comprising the steps:
- exposing the bulk material which is preferably in its desired final form to a low pressure plasma in a vapor of at least one organic and/or inorganic compound under conditions whereby a thin film containing reactive groups is deposited on the suface of said bulk material,
- optionally reacting the said reactive groups with an activating group, and/or with an oligofunctional compound having at least two functional groups capable of chemically reacting with the said reacting groups, or with the activated reactive groups, having at least one additional functional group capable of chemically reacting with a hydrophilic coating material such as a polysaccharide,
- reacting the reactive groups or the functional groups on the surface with a hydrophilic coating material such as a polysaccharide,
- treating said surface-immobilized hydrophilic coating material with a neutralizing agent, in particular with a charge neutralizing agent, which is selected from an organic acid and/or an organic base.

14. A process for the manufacture of a composite material with a wettable surface being significantly less susceptible to protein absorption (fouling) in accordance to claim 1, wherein reactive groups are inherently present in the bulk material, said process comprising the steps:
- optionally reacting the reactive groups inherently present in the bulk material with an activating group, and/or with an oligofunctional compound having at least two functional groups capable of chemically reacting with the said reacting groups, or with the activated reactive groups, having at least one additional functional group capable of chemically reacting with a hydrophilic coating material such as a polysaccharide,
- reacting the reactive groups or the functional groups on the surface with a hydrophilic coating material such as a polysaccharide,
- treating said surface-immobilized hydrophilic coating material with a neutralizing agent, in particular with a charge neutralizing agent, which is selected from an organic acid and/or an organic base.
